(19) **Europäisches Patentamt / European Patent Office / Office européen des brevets**

(11) **EP 4 386 061 B1**

(12) **EUROPEAN PATENT SPECIFICATION**

(45) Date of publication and mention
of the grant of the patent:
**02.07.2025  Bulletin 2025/27**

(21) Application number: **22213825.7**

(22) Date of filing: **15.12.2022**

(51) International Patent Classification (IPC):
*C09J 167/04* (2006.01)    *C08G 63/91* (2006.01)

(52) Cooperative Patent Classification (CPC):
**C08G 63/912; C09J 167/04; C12P 7/625**

(54) **POLYHYDROXYALKANOATE - BASED PRESSURE-SENSITIVE ADHESIVE**

DRUCKEMPFINDLICHES HAFTMITTEL AUF POLYHYDROXYALKANOATBASIS

ADHÉSIF SENSIBLE À LA PRESSION À BASE DE POLYHYDROXYALCANOATE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**19.06.2024  Bulletin 2024/25**

(73) Proprietor: **tesa SE
22848 Norderstedt (DE)**

(72) Inventors:
• **ROGGE, Poom
  20146 Hamburg (DE)**
• **WEGENER, Philipp
  21614 Buxtehude (DE)**
• **JUNGHANS, Monika
  22457 Hamburg (DE)**

(74) Representative: **tesa SE
Hugo-Kirchberg-Straße 1
22848 Norderstedt (DE)**

(56) References cited:
**WO-A1-2023/275619    WO-A2-2019/092118**

## Description

The invention relates to the technical field of pressure-sensitive adhesives. Specifically, the invention proposes a particularly sustainable pressure-sensitive adhesive essentially based on functionalized mcl polyhydroxyalkanoates (PHAs), the side chains of which are functionalized by bulky groups. It has been found that such polyhydroxyalkanoates have a low degree of crystallinity and allow for the preparation of pressure-sensitive adhesives that adhere well to a variety of substrates and are biologically degradable.

Adhesive tapes which are furnished with pressure-sensitive adhesives, known as pressure-sensitive adhesive (PSA) tapes, are nowadays diversely used in the industrial and private spheres. PSA tapes consist customarily of a carrier film, furnished on one or both sides with a PSA. There are also PSA tapes consisting exclusively of a layer of PSA and no carrier film, such tapes being called transfer tapes. The composition of the PSA tapes may vary greatly and is guided by the particular requirements of the various applications. The carriers consist customarily of polymeric films such as, for example, polypropylene, polyethylene, polyester, or else of paper or woven or nonwoven fabric.

The PSAs consist customarily of acrylate copolymers, silicones, natural rubber, synthetic rubber, styrene block copolymers, or polyurethanes.

On the basis of environmental considerations and sustainability, and against the background of increasingly scarce petroleum resources, and, on the other hand, a sharp worldwide growth in consumption of plastics, there have been efforts for a number of years to produce plastics on the basis of renewable raw materials. This is especially true of biodegradable polymers which are intended for use in packaging applications or film applications. For medical applications as well, biodegradable products are playing an increasingly important role. Certain biobased or biodegradable plastics are available commercially today. Biobased means "produced from renewable raw materials".

The term "biodegradable polymers" is used for natural and synthetic polymers which have plastics-like qualities (notched impact strength, thermoplastifiability) but which, in contrast to conventional plastics, are degraded by a multiplicity of microorganisms in a biologically active environment (compost, digested sludge, soil, wastewater); this does not necessarily take place under customary household conditions (garden composting). One definition of biodegradability is given in European Standard DIN EN 13432 (biodegradation of packaging) and DIN EN 14995 (compostability of plastics).

The skilled person makes a distinction between disintegration and biodegradability.

Disintegration refers to physical breakdown into very small fragments.

Determining the disintegrability (the degree of disintegration) of polymers is described in texts including DIN EN ISO 20200. In that case, the sample under analysis is stored in a defined artificial solids waste system at $58 \pm 2°C$ for at least 45 and not more than 90 days. Thereafter the entire sample is passed through a 2 mm sieve and the degree of disintegration D is determined as follows:

$$D = \frac{m_i - m_r}{m_i} \times 100$$

Here

mi is the initial dry mass of the sample material
and

mr is the dry mass of the residual sample material obtained by sieving.

Biodegradability is understood in general as the capacity for disintegration of a chemical compound or of an organic material by microorganisms in the presence of oxygen into carbon dioxide, water, and salts of other elements present (mineralization), with formation of new biomass, or, in the absence of oxygen, into carbon dioxide, methane, mineral salts, and new biomass. Biodegradation is accomplished outside and/or inside the cell by bacteria, fungi, and microorganisms, and also their enzymes.

The biodegradability of packing materials is governed in terms of standards by DIN EN 13432 "Requirements for packaging recoverable through composting and biodegradation". In this context, the material for testing is subjected to an aerobic degradation test in accordance with ISO 14855:1999 "Determination of the ultimate aerobic biodegradability of plastic materials under controlled composting conditions" and a degree of degradation of at least 90% must be achieved in comparison to a suitable reference substance within not more than six months. The degree of degradation here is determined by the measured evolution of carbon dioxide. After comminution, the sample is stored with vermiculite or highly functional aerated compost as inoculum in a vessel equipped with air supply at $58 \pm 2°C$, and the evolution of $CO_2$ is recorded on an ongoing basis. In view of the complexity of apparatus, there are a number of testing institutes which have specialized in the testing and which subsequently issue a corresponding certificate, such as, for instance, OK compost from Vingotte.

After the end of the testing, the degradation rate $D_t$ is obtained as:

$$D_t = \frac{(CO_2)_T - (CO_2)_B}{ThCO_2} \times 100$$

Here

$(CO_2)_T$ is the cumulative amount of carbon dioxide formed in each composting vessel containing the test substance, in grams per vessel;

$(CO_2)_B$ is the average cumulative amount of carbon dioxide formed in the control vessels, in grams per vessel;

$ThCO_2$ is the theoretical amount of carbon dioxide which the test substance can form, in grams per vessel.

In addition to the biodegradability, DIN EN 13432 also includes a test for determining the quality of the compost produced as a result of degradation. This compost must not have any adverse effects on plant growth.

Generally speaking, biodegradable components also have a high disintegration rate, whereas the disintegration of a material does not automatically imply its biodegradability.

In view of the fact that environmental considerations relating to biodegradability are playing an ever more important part not least for pressure-sensitive adhesive tapes, the past has also seen the presentation of PSA tapes which use biodegradable films as carrier material. The films used are frequently based on compounds of polylactic acid. Like other biodegradable thermoplastic polymers suitable for application, polylactic acid is relatively hard and brittle. In order to be suitable for film applications, these biodegradable polymers contemplated must be compounded with softer polymers, which in many cases lack or have poorer biodegradability.

In terms of the PSAs, the possibilities are further restricted. PSAs are amorphous materials with a low glass transition point.

The conventional scaffold polymers such as natural rubber, styrene block copolymers or polyacrylates are not biodegradable in accordance with the standards which apply in Europe, such as DIN EN 13432, for example. The same is true of the customary tackifier resins such as rosin derivatives, hydrocarbon resins or terpene-phenolic resins. Silicone PSAs are out of the question entirely, on account of their excellent stability with respect to aging. A criterion of biodegradability is customarily the presence of heteroatoms in the main carbon chain. A chemical bond between a carbon atom and a heteroatom such as oxygen or nitrogen, for example, is less stable and hence more amenable to biological degradation than is a bond between two carbon atoms.

Against this background and due to their biological origin and structural variety, polyhydroxyalkanoates (PHAs) potentially offer a promising approach towards sustainable basic polymers for biologically degradable pressure-sensitive adhesives. However, to seriously qualify as a base material, several criteria have to be met:

A) sufficiently high molecular weight;
B) low Glass Transition Temperature (Tg);
C) amorphous character of the material; and
D) high entanglement molar mass.

In this respect, due to their biological origin and high tacticity, most of the known PHAs readily crystalize and are thus unsuitable for the formulation of pressure-sensitive adhesives.

WO 2019/092118 A2 discloses an adhesive formulation, e.g. a pressure-sensitive adhesive, that comprises 5 to 98 wt.-% biodegradable, non-crystalline mcl-PHA. The mcl-PHA can be cured with a peroxide curing agent. The adhesive formulation may include an additional biodegradable polymer or a non-biodegradable thermoplastic elastomer.

US 2018/0186928 A1 discloses a process of forming a side-chain-functionalized polyhydroxyalkanoate material. The process includes forming a PHA material having a hydroxy-terminated side chain. The process also includes utilizing the PHA material having the hydroxy-terminated side chain to form a side chain-functionalized PHA material having a side chain with a terminal crosslinkable functional group.

It was an objective of the invention to provide a substantially biodegradable pressure-sensitive adhesive being to a large extent based on materials of biological origin.

It was a further objective of the invention to combine the sustainability features of the pressure-sensitive adhesive with good tack and good adhesion properties on a variety of substrates, among them human skin. It was furthermore desired that the pressure-sensitive adhesive securely adheres to the substrate even when the substrate is exposed to external impacts like water or - in case of the substrate being skin - perspiration or even shower gel.

It was a further objective of the invention to provide a pressure-sensitive adhesive that is easily removable and repositionable without substantially losing its tackiness and adhesive properties after having been initially attached to a substrate.

In order to solve these problems, a first and common subject of the invention is a pressure-sensitive adhesive comprising a polyhydroxyalkanoate (PHA), characterized in that the polyhydroxyalkanoate features 1 to 20 weight-% of structural units having a side chain functionalization moiety comprising at least 10 C-atoms, a side chain functionalization moiety being a chemical group provided by further functionalization of the original side chain originating from the hydroxy alkane acids on which the PHA is formally based; wherein the side chain functionalization moiety is bonded to the side chain via a heteroatom.

Pressure-sensitive adhesiveness is that property of a substance whereby it enters into a permanent bond to a substrate even under relatively weak applied pressure. Substances possessing this quality are identified as pressure-sensitive adhesives (PSAs). PSAs are long-established products. Frequently they can be detached from the substrate again after use, with substantially no residue. PSAs generally at room temperature have a permanent inherent stickiness, thus having a certain viscosity and tack, and so they wet the surface of the respective substrate even under low applied pressure. The capacity of a PSA to adhere to materials and to transmit forces derives from the adhesion capacity and the cohesion of the PSA.

PSAs can be viewed as liquids with extremely high viscosity with an elastic component. Accordingly, PSAs have particular, characteristic viscoelastic properties which result in the permanent inherent tack and adhesiveness.

A characteristic of PSAs is that when they are mechanically deformed, there are processes of viscous flow and there is also development of elastic forces of resilience. The two processes have a certain relationship to one another in terms of their respective proportion, in dependence not only on the precise composition, the structure, and the degree of crosslinking of the respective PSA but also on the rate and duration of the deformation, and on the temperature.

The proportional viscous flow is necessary for the achievement of adhesion. Only the viscous components, brought about by macromolecules with relatively high mobility, permit effective wetting and flow onto the substrate where bonding is to take place. A high viscous flow component results in high tack (also referred to as surface stickiness) and hence often also in high peel adhesion. Highly crosslinked systems, crystalline polymers, or polymers with glasslike solidification lack flowable components and are therefore in general devoid of tack or at least possess only little tack.

The proportional elastic forces of resilience are necessary for the attainment of cohesion. They are brought about, for example, by very long-chain macromolecules with a high degree of coiling, and also by physically or chemically crosslinked macromolecules, and they permit the transmission of the forces that act on an adhesive bond. As a result of these forces of resilience, an adhesive bond is able to withstand a long-term load acting on it, in the form of a long-term shearing load, for example, sufficiently over a relatively long time period.

For the more precise description and quantification of the extent of elastic and viscous components, and also of the proportion of the components to one another, the variables of storage modulus (G') and loss modulus (G") can be employed, as may be determined by means of Dynamic Mechanical Analysis (DMA). G' is a measure of the elastic component, G" a measure of the viscous component, of a substance. Both variables are dependent on the deformation frequency and on the temperature.

The variables can be determined with the aid of a rheometer. In that case, for example, the material under investigation, in the form of a plane-parallel layer, is exposed in a plate/plate arrangement to a sinusoidally oscillating shearing stress. In the case of instruments operating with shear stress control, the deformation is measured as a function of time, and the time offset of this deformation relative to the introduction of shear stress is recorded. This time offset is referred to as phase angle $\delta$.

The storage modulus G' is defined as follows: $G' = (\tau/\gamma) * \cos(\delta)$ ($\tau$ = shear stress, $\gamma$ = deformation, $\delta$ = phase angle = phase shift between shear stress vector and deformation vector). The definition of the loss modulus G" is as follows: $G" = (\tau/\gamma) * \sin(\delta)$ ($\tau$ = shear stress, $\gamma$ = deformation, $\delta$ = phase angle = phase shift between shear stress vector and deformation vector).

A substance and the layer produced from it are in particular deemed to be pressure-sensitively adhesive if at room temperature, here by definition at 23 °C, in the deformation frequency range from $10^0$ to $10^1$ rad/sec, G' and G" are each situated at least partly in the range from $10^3$ to $10^7$ Pa.

Within this range, which in a matrix plot of G' and G" (G' plotted as a function of G") may also be referred to as a viscoelastic window for PSA applications or as a PSA window according to viscoelastic criteria, there are in turn different sectors and quadrants which characterize more closely the PSA properties to be expected from the associated substances. Within this window, substances with high G" and low G' are generally notable, for example, for a high peel adhesion and a low shear strength, whereas substances with high G" and high G' are notable both for high peel adhesion and for high shear strength.

The findings on the correlations between rheology and pressure-sensitive adhesiveness, generally, are state of the art and are described for example in Satas, "Handbook of Pressure Sensitive Adhesive Technology", Third Edition (1999), pages 153 to 203.

Polyhydroxyalkanoates, abbreviated PHAs, are polyesters which can formally be traced back to a monomer basis formed by one or mor hydroxy alkane acids. Accordingly, they have a structure of the formula $H-[-O-R-C(O)-]_n$, wherein R is an alkylene group that can generally be branched or non-branched and functionalized or non-functionalized. In general, however, PHAs are not synthesized by way of polymerization of hydroxy alkane acids but are biologically produced by bacterial cultures. The composition of the PHAs is determined therein by the biological material that is exposed to the bacteria and in particular by the selection of the bacteria.

The polyhydroxyalkanoate of the pressure-sensitive adhesive according to the invention preferably comprises at least 50 wt.-% of structural units having a side chain protruding from the polymer main chain and having 3 to 9 C-atoms. More preferably, the PHA comprises such structural units in an amount of at least 60 wt.-%, particularly preferably of at least 70 wt.-%, even more preferably of at least 80 wt.-% and most preferably of at least 90 wt.-%. Accordingly, the PHA preferably is formally based on at least 50, at least 60, at least 70, at least 80 or at least 90 wt.-% on hydroxy alkane acids having no terminal OH group but having in their structural sequence following the hydroxy group and pointing away from the carboxy group 3 to 9 C-atoms. The polymer chain formally originating from the reaction of the carboxy groups with the hydroxy groups accordingly has $C_3$-$C_9$ alkyl groups that protrude from the main chain.

Particularly preferably the polyhydroxyalkanoate comprises at least 60 wt.-%, at least 70 wt.-%, at least 80 wt.-% or at least 90 wt.-% structural units that have a side chain protruding from the main chain, the side chain having 4 to 8, more preferably 4 to 7 C-atoms.

Preferably, the hydroxy alkane acids on which the polyhydroxyalkanoate of the pressure-sensitive adhesive according to the invention is formally based are hydroxylated in 3- or 4-position. Particularly preferably, the PHA comprises at least 50, at least 60, at least 70, at least 80 or at least 90 wt.-% of structural units that can be traced back to one or more hydroxy alkane acids selected from the group consisting of 3-hydroxy hexane acid, 3-hydroxy heptane acid, 3-hydroxy octane acid, 3-hydroxy nonane acid, 3-hydroxy decane acid, 3-hydroxy undecane acid, 3-hydroxy dodecane acid, 4-hydroxy heptane acid, 4-hydroxy octane acid, 4-hydroxy nonane acid, 4-hydroxy decane acid, 4-hydroxy undecane acid and 4-hydroxy dodecane acid. PHAs based on such acids are commonly referred to as medium chain length PHAs (mcl-PHAs).

Particularly preferably, the PHA comprises at least 50, at least 60, at least 70, at least 80 or at least 90 wt.-% of structural units that can be traced back to one or more hydroxy alkane acids selected from the group consisting of 3-hydroxy heptane acid, 3-hydroxy octane acid, 3-hydroxy nonane acid, 3-hydroxy decane acid, 3-hydroxy undecane acid, 4-hydroxy octane acid, 4-hydroxy nonane acid, 4-hydroxy decane acid, 4-hydroxy undecane acid and 4-hydroxy dodecane acid.

The production of medium chain length polyhydroxyalkanoates is described e.g. in Appl. Microbiol. Biotechnol. 2014, Jan; 98(2): 611-620. Epoxidized mcl-PHAs are generally known from e.g. "Modification of polyhydroxyalkanoates"", January 2015 - RSG Green Chemistry 2015 (30); 141-182.

Beyond the structural units set forth hereinbefore, the polyhydroxyalkanoate may further comprise structural units on which short chain length PHAs are based, e.g. structural units that can be traced back to 3-hydroxy butyric acid, 4-hydroxy butyric acid (4-HB), 3-hydroxy valeric acid, 4-hydroxy valeric acid (4-HV) and/or 4-hydroxy hexane acid (4-HX). Thus, structural units having no side chain functionalization and/or further methyl or ethyl side chains protruding from the main chain may be comprised.

According to the invention, the PHA features 1 to 20 wt.-% of structural units having a side chain functionalization moiety comprising at least 10 C-atoms. Preferably, the PHA features 3 to 17 wt.-% and most preferably 7 to 13 wt.-% of structural units having a side chain functionalization moiety comprising at least 10 C-atoms. By "side chain functionalization moiety" a further chemical group is meant that does not form part of the original side chain originating from the hydroxy alkane acids on which the PHA is formally based. Rather, the "side chain functionalization moieties" are provided by further functionalization of these side chains. Preferably, the side chains are modified by way of chemical reaction which in turn means that the side chains need to comprise reactive groups. Such reactive groups may e.g. be provided by adding acids having a side chain terminated with a reactive group to the acids that are exposed to the bacterial cultures.

As an example, fatty acids having an olefinic double bond in their chain may be added to the composition from which the PHA is produced, e.g. fermented. Thus, PHAs comprising side chains having an olefinic double bond may be produced. The double bond-containing side chains may then be subject to further functionalization, e.g. by way of intermediately forming hydroxy-containing groups from the olefinic double bond structures or by epoxidation of the double bonds and further reaction with appropriate functionalizing substances. Accordingly, the side chain functionalization moiety is bonded to the side chain via a heteroatom.

The side chain functionalization moiety is bonded to the side chain via a heteroatom. Particularly preferably, the heteroatom is selected from the groups consisting of N-, O-, Si-. Sand Si-atoms; most preferably, the heteroatom is an O-atom.

Preferably, the side chain functionalization moiety is branched. More preferably, the side chain functionalization moiety is a bulky or, in other words, sterically demanding group. It is therefore preferred if the side chain functionalization moiety comprises at least 12, at least 15 and particularly at least 18 C-atoms.

Further preferably, the side chain functionalization moiety comprises at least two, more preferably at least three and particularly preferably at least four C-atoms that are chirality centers, i.e. they are asymmetric C-atoms that thus carry four different substituents. Thereby, sterically demanding structures are formed which advantageously seem to support the amorphous character of the PHAs of the pressure-sensitive adhesive according to the invention.

Also preferably, the side chain functionalization moiety comprises at least two, more preferably three joint ring structures. A "joint ring structure" consists of two rings that are joint by at least one covalent bond that forms part of both rings. Joint ring structures also advantageously contribute to sterically demanding functionalization moieties.

Preferably, the side chain functionalization moiety is derived from one or more naturally occurring substances. More preferably, the side chain functionalization moiety is derived from one or more naturally occurring acids. Highly preferably, the side chain functionalization moiety is derived from one or more resin acids. Resin acids, also referred to as rosin acids, form the main component of wood resin or rosin, respectively. As such, the resin acids are preferably selected from the group consisting of abietic acid, dihydroabietic acid, tetrahydroabietic acid, dehydroabietic acid, neoabietic acid, pimaric acid, levopimaric acid and parastrinic acid. Most preferably, the main constituent of the side chain functionalization moiety is derived from abietic acid.

In an embodiment of the invention, the side chain functionalization moiety is provided via a method comprising the steps of

a) forming a PHA having side chains comprising an olefinic double bond by addition of fatty acids to a composition for preparing a PHA and exposing the composition to bacterial fermentation;
b) epoxidation of at least a part of the olefinic double bonds thus introduced into the PHA;
c) reacting the resulting PHA having epoxide-containing side chains with an acid having at least 10 C-atoms.

The olefinic double bonds mentioned in steps a) and b) preferably are terminal olefinic double bonds. Thus, the method according to the embodiment most preferably comprises the steps of

a) forming a PHA having vinyl-terminated side chains by addition of vinyl-terminated fatty acids to a composition for preparing a PHA and exposing the composition to bacterial fermentation;
b) epoxidation of at least a part of the terminal vinyl groups thus introduced into the PHA;
c) reacting the resulting PHA having epoxide-terminated side chains with an acid having at least 10 C-atoms.

In accordance with what is set forth above, the acid having at least 10 C-atoms as used in step c) preferably is a naturally occurring acid. More preferably, the acid is a resin acid or rosin acid, respectively and is as such preferably selected from the group consisting of abietic acid, dihydroabietic acid, tetrahydroabietic acid, dehydroabietic acid, neoabietic acid, pimaric acid, levopimaric acid and parastrinic acid. Most preferably, the acid is abietic acid.

Preferably, the pressure-sensitive adhesive according to the invention comprises one or more polyhydroxyalkanoate as described hereinbefore in a total amount of 30 to 90 wt.-%, more preferably in a total amount of 40 to 80 wt.-%, particularly preferably in a total amount of 50 to 70 wt.-%, most preferably in a total amount of 55 to 65 wt.-%, based on the total weight of the pressure-sensitive adhesive.

The pressure-sensitive adhesive according to the invention may further comprise one or more substances that may impart plasticizing properties to the adhesive. In relation thereto, the pressure-sensitive adhesive according to the invention preferably comprises at least one substance selected from the group consisting of dialkyl adipates, e.g. dioctyl adipate; polyadipates; poly[di(ethylene glycol)]; tri(ethylene glycol); bis(2-ethylhexanoate); dialkyl phthalates, e.g. dioctyl phthalate and dibutyl phthalate; glycerol; glycerol triacetate; salicylic acid; aliphatic di- and polyacid based esters, e.g. sebacic acid esters, adipic acid esters, citric acid esters; 1,2-cyclohexanedicarboxylic acid diisononyl ester; oils, e.g. soybean oil and epoxidized soybean oil; dodecanol; lauric acid; tributyrin; trilaurin and polyethylene glycol. More preferably, the pressure-sensitive adhesive comprises at least one substance selected from aliphatic di- and polyacid based esters, in particular selected from sebacic acid esters, adipic acid esters and citric acid esters. Most preferably, the pressure-sensitive adhesive comprises at least one citric acid ester, in particular acetyl tri-n-butyl citrate. One or more of the compounds as described hereinbefore are preferably comprised by the pressure-sensitive adhesive according to the invention in total amounts of 1 to 15 wt.-%, more preferably in total amounts of 3 to 10 wt.-%, most preferably in total amounts of 4 to 8 wt.-%, based on the total weight of the pressure-sensitive adhesive.

Furthermore, the pressure-sensitive adhesive according to the invention preferably comprises at least one sugar ester. More preferably, the pressure-sensitive adhesive comprises at least one sucrose ester. In particular, the pressure-sensitive adhesive comprises at least one aromatic sucrose ester. Most preferably, the pressure-sensitive adhesive comprises sucrose benzoate.

Preferably, the pressure-sensitive adhesive according to the invention comprises sugar ester in a total amount of 10 to 50 wt.-%, more preferably in a total amount of 15 to 40 wt.-% and most preferably in a total amount of 18 to 35 wt.-%.

Preferably, the PHA of the pressure-sensitive adhesive according to the invention is crosslinked. Preferably, crosslinking of the PHA is effected by one or more crosslinkers selected from the group consisting of carbodiimides, diols, polyols, diisocyanates, polyisocyanates, isocyanurates, phenol formaldehyde resins, urea formaldehyde resins, melamine formaldehyde resins, diamines, polyamines, dithiols, polythiols, dianhydrides, polyanhydrides, epoxides, acids, bases and redox systems.

Alternatively or - preferably - additionally, the PHA can be radically crosslinked. Radical crosslinking can be initiated by thermal and/or UV initiators. Preferred initiators are Azo systems like VAZO® 67; redox systems like APS or KPS with TEMED; and peroxides. Preferably, radical crosslinking of the PHA is effected by peroxides, especially diperoxides; most preferably, radical crosslinking of the PHA is effected by dibenzoyl peroxide.

Preferably, the PHA is radically crosslinked by means of an initiator and at least one, more preferably at least two, coagents. The coagent is preferably selected from the group consisting of sulfide compounds, in particular tetraethyl thiuram disulfide (TEDS), tetramethyl thiuram disulfide (TMTDS), bis(piperdinothiocarbonyl) disulfide (BPTDS) and 2,2¢-dithiobis(benzothiazole) (DTBzT); multifunctional coagents, in particular triallyl isocyanurate (TAIC), triallyl trimester (TAM), trimetylolproprane triacrylate (TMPTA), triallyl cyanurate (TAC), pentaerythritol triacrylate (PETA).

Most preferably, the coagent is selected from sulfide compounds, in particular tetraethyl thiuram disulfide (TEDS), tetramethyl thiuram disulfide (TMTDS), bis(piperdinothiocarbonyl) disulfide (BPTDS) and 2,2'-dithiobis(benzothiazole) (DTBzT). These compounds have proven to prevent beta-scission of the PHA and further to improvei the effectiveness of the crosslinking.

Further, the PHA can be crosslinked via UV crosslinking.

In addition to the components set forth hereinbefore, the pressure-sensitive adhesive according to the invention may comprise further additives, e.g. UV absorbers, antioxidants, tackifiers, fillers, pigments, dyes, flame retardants, foaming substances, tensides etc.

A further subject matter of the invention is an adhesive tape comprising the pressure-sensitive adhesive according to the present invention. In particular, the adhesive tape can have one or two layers of the pressure-sensitive adhesive according to the invention as its adhesive layers. The adhesive layers may be covered by a release liner as generally known in the art.

The adhesive tape may further comprise a backing which may be a film, a woven, a non-woven or a paper backing. In particular, the backing may be a polyhydroxyalkanoate film, a polybutylene succinate film, a polybutylene adipate terephthalate film, a polybutylene terephthalate film and a cellophane film.

Experimental Section

Methods:

Gel Permeation Chromatography GPC (Method A):

The figures for molecular weights and polydispersity D in this document were determined by gel permeation chromatography. The measurement was carried out on 50 μl filtered sample (sample concentration approx. 3 g/l). Tetrahydrofuran was used as eluent. The measurement was carried out at 25 °C. An SDV column with the specifications 5 μm, ID 8.0 mm x 50 mm, was used. For separation, a column combination of three SDV columns (5 μm, ID 8.0 mm x 300 mm) with individual porosities of $10^3$ Å, $10^5$ Å and $10^6$ Å (columns of the company Polymer Standards Service) were used; Detection by differential refractometer PSS-SECurity 1260). The flow rate was 1 ml/min. Calibration was performed against PS (relative polystyrene calibration) standards.

Adhesion (Method B):

B1- Peel-Adhesion on stainless-steel:
The determination of the adhesive peel adhesion was carried out at a test condition of 23 °C +/- 1 °C temperature and 50 % +/- 5 % relative humidity. The adhesive samples on an etched PET film of 36 μm thickness were cut to a width of 20 mm and attached to a stainless-steel plate. The stainless-steel plate was cleaned and conditioned before measurement by wiping 4 times with acetone and then leaving in air for 10 minutes so that the solvent could evaporate. The test sample was then rolled onto the steel substrate. The adhesive was rolled back and forth four times with a 2-kg roll at a winding speed of 0.6 m/min. The plate was pushed into a special holder that allows the sample to be peeled off at an angle of 180°. The adhesive peel adhesion measurement was carried out with a Zwick tensile testing machine at a speed of 300 mm/min. The measurement results are given in N/cm and are averaged from three individual measurements.

B2- Peel-Adhesion on Skin:
The method is based on DIN EN 1939, which describes the measurement of the adhesive strength on metal or textured surface (double-sided coated adhesive tape) at a specified temperature and peel-angle. The in vivo examination of the adhesive was carried out on human skin. The measurements were performed in a room with controlled conditions (23°C +/- 2°C; humidity 50% +/- 5%) on the forearms. Before starting the measurement, the forearms were carefully cleaned to remove creams, lotions, and similar substances. On each forearm, 3 (100 mm x 13 mm) strips (adhesive is coated previously on a textile backing) were applied and worn for 1 hour before being peeled off at a 90° angle. The adhesive strength measurement was carried out with a Zwick tensile testing machine at a speed of 300 mm/min. The measurement results are given in N/cm and are averaged from three individual measurements.

Microshear test (MSW, Method C):

Based on ASTM D 4498, the procedure is as follows: A sample of the adhesive was equipped on one side with an etched PET film of 36 $\mu$m thickness for stabilization. A test strip 10 mm wide and 50 mm long was attached to a clean stainless-steel plate in such a way that a bonding surface of 130 mm$^2$ results. The sample was rolled back and forth three times with a 2 kg roll. The stainless-steel plate was adjusted in the measuring apparatus so that the test strip was in vertical position. The system was heated to 40 °C. At the start of the measurement, a weight of 100 g was attached to the free end of the test strip by means of a clamp (6.4 g net weight), which loads the sample by gravity; At the same time, a micrometer probe was placed on a short piece of the test adhesive strip protruding over the stainless-steel plate, which registered the dislocation depending on the measuring time, i.e., the shearway was graphically recorded. Micro shear displacement S1 (maximum micro shear displacement) was the shear distance after a weight load of 15 min. After this measurement time, the weight was carefully removed from the sample and then relaxation was observed for another 15 minutes. After this relaxation time, the micro shear path S2 (relaxed micro shear path) was determined. From the two measured values, the micro shear displacement quotient E = S2/S1 (elastic part) was determined. This quotient E is a measure of the elasticity (restoring force) of the pressure-sensitive adhesive. The difference V = 1-E (viscous part) is a measure of the inelastic flow properties of the adhesive.

Glass Transition Temperature Tg (Method D):

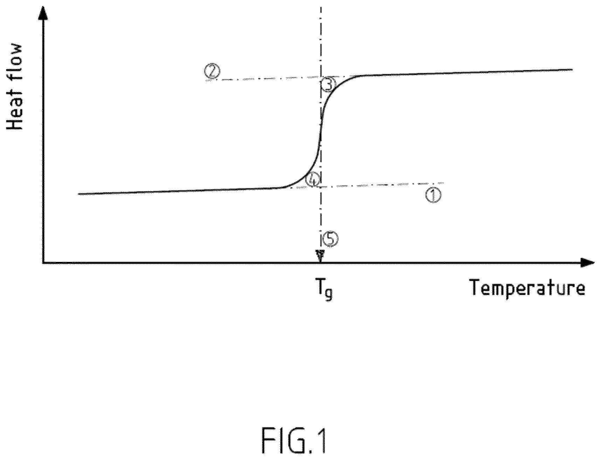

The static glass transition temperature ($T_g$) was determined by means of dynamic scanning calorimetry (DSC). For this purpose, about 5 mg of an untreated polymer sample is weighed into an aluminum boat (volume 25 $\mu$l) and closed with a punctured lid. The measurement is made using a DSC 204 F1 from Netzsch. A nitrogen atmosphere is employed for inertization. The sample is first cooled down to -150°C, then heated up to +150°C at a heating rate of 10 K/min and cooled down again to -150°C. The subsequent second heating curve is run again at 10 K/min and the changing heat capacity is recorded. Glass transitions are recognized as steps in the thermogram.
Therein, the glass transition temperature is obtained as follows (see figure 1):
The respective linear region of the measurement curve before and after the step is extended in the direction of rising temperatures (area before the step) or falling temperatures (area after the step) (extension lines ① and ②). In the region of the step, a line of best fit (⑤) is run parallel to the ordinate such that it intersects with both extension lines, specifically in such a way as to form two equal areas ③ and ④ (between the respective extension line, the line of best fit and the measurement curve). The point of intersection of the line of best fit thus positioned with the measurement curve gives the glass transition temperature.

Shear Strength (Method E):

Tests were conducted on adhesive strips applied to a stainless-steel plate such that the adhesion area of 13 mm by 20 mm was in contact with the plate. Then the adhesive tape was rolled on 3 times back and forth with a 2 kg roller. After the rolling, a belt loop was attached to the end of the adhesive tape. A force of one kilogram was applied as a hanging weight on the belt loop. The time elapsed for each test specimen to separate from the stainless-steel plate was recorded as the shear strength. The time at which the mass falls is the "Shear Test" result. If the tape has not failed after 10000 minutes, the result is reported as 10000+. Three specimens of each tape were tested and the shear tests were averaged to obtain the shear strength value.

Laboratory disintegration test (Method F)

A container made of polypropylene was used in the experiment.

Compost was mixed with water to achieve a final water content of 55% in total. 150 g of the compost was put into the container. 1.5g of the adhesive was put on the compost. Another 150 g of the compost was added on top of the adhesive and the first compost section.

A lid with two holes of 5 mm diameter each was used to cover the container to avoid excessive evaporation and to provide gas exchange. The sample container was stored at 58 ± 2 °C for 90 days. The water content was maintained twice a week to restore the initial mass, if needed.

At the end of the test, the container was removed from the oven to check the degree of disintegration. The sample passed the disintegration test if at least 90 % of the adhesive sample fragmented to particles < 2 mm in size within 90 days.

**Examples:**

Preparation of amorphous mcl PHA

Starting point is a mcl PHA containing olefin groups in the side chains (10 %) provided by TerraVerdae Bioworks Inc. (mPHA-1110). This polymer is reacted with meta-chloroperbenzoic acid (mCPBA) in a 1:2 molar ratio (olefinic groups : reactive oxygen) in ethyl acetate and refluxed for 4 hours at 100°C to obtain an epoxidized mcl PHA (mPHA-1110E).

Step 2: mPHA-1110E is dissolved with rosin acids (Foral AX-E) in a 1:1 wt.-ratio in acetone. After polymer and gum rosin completely dissolved, triethylamine (TEA) was added as a catalyst, and mixing was continued for 30 minutes. Then the solvent was partially removed and the remaining content was heated at 120 °C for 24 h to obtain amorphous PHA (mPHA-am1).

Preparation of pressure-sensitive adhesive

Sample 1 - on sensitive backing

mPHA-am1 was dissolved in acetone to yield a solids content of 27.27 wt.-%. Sucrose benzoate and Citroflex A4 were added to the polymer solution. The solution was stirred with a magnetic stirrer at room temperature for 2 hours to yield a homogeneous mixture. Dibenzoyl peroxide, triallyl isocyanurate and tetraethylthiuram disulfide were added. The solution was stirred with a magnetic stirrer at room temperature for 1 hour to yield a homogeneous mixture. The homogeneous mixture was coated on a PET process liner with a coating bar to achieve a thickness of 50 $\mu$m. A non-woven, polyester textile backing was applied on the adhesive sheet by rolling with light pressure to ensure that the backing attaches to the adhesive. The adhesive sheet thus obtained was dried at 150 C for 10 minutes.

Sample 2 - on etched PET

mPHA-am1 was dissolved in acetone to yield a solids content of 27.27 wt.-%. Sucrose benzoate and Citroflex A4 were added to the polymer solution. The solution was stirred with a magnetic stirrer at room temperature for 2 hours to yield a homogeneous mixture. Dibenzoyl peroxide, triallyl isocyanurate and tetraethylthiuram disulfide were added. The solution was stirred with a magnetic stirrer at room temperature for 1 hour to yield a homogeneous mixture. The homogeneous mixture was coated on an etched PET backing using a coating bar. The adhesive sheet thus obtained was dried at 150 C for 10 min. The final thickness of the adhesive was 50 $\mu$m.

Table 1: Composition of the pressure-sensitive adhesive

| Components in wt.-% | 2.1 | 2.2 | 2.3 |
|---|---|---|---|
| mPHA-am1 | 60.6 | - | - |
| mPHA-1110E | - | 64.9 | 60.6 |
| Sucrose benzoate | 26.0 | 27.8 | 26.0 |
| Citroflex A4 | 6.7 | 7.3 | 6.7 |
| Dibenzoyl peroxide (BPO) | 3.2 | - | 3.2 |
| Triallyl isocyanurate (TAIC) | 3.2 | - | 3.2 |
| Tetraethylthiuram disulfide (TEDS) | 0.3 | - | 0.3 |

Table 2: Test results

| Sample | Peel adhesion (Method B1; N/cm) | Peel adhesion (Method B2; N/cm) | Disintegration test (Method F) |
|---|---|---|---|
| 2.1 | 4.19 | 1.71* | Passed |
| 2.2 | 1.50 | - | Passed |
| 2.3 | 0.20 | - | Passed |
| *Standard skin adhesives for medical devices show values between 0.2 and 2 N/cm. | | | |

## Claims

1. Pressure-sensitive adhesive comprising

   a polyhydroxyalkanoate, **characterized in that** the polyhydroxyalkanoate features 1 to 20 weight-% of structural units having a side chain functionalization moiety comprising at least 10 C-atoms, a side chain functionalization moiety being a chemical group provided by further functionalization of the original side chain originating from the hydroxy alkane acids on which the PHA is formally based;
   wherein the side chain functionalization moiety is bonded to the side chain via a heteroatom.

2. Pressure-sensitive adhesive according to claim 1, **characterized in that** the heteroatom is an O-atom.

3. Pressure-sensitive adhesive according to any of the preceding claims, **characterized in that** the side chain functionalization moiety is branched.

4. Pressure-sensitive adhesive according to any of the preceding claims, **characterized in that** the side chain functionalization moiety is derived from a naturally occurring substance.

5. Pressure-sensitive adhesive according to any of the preceding claims, **characterized in that** the side chain functionalization moiety comprises at least one ring structure.

6. Pressure-sensitive adhesive according to any of the preceding claims, **characterized in that** the side chain functionalization moiety comprises at least two C-atoms which are chirality centers.

7. Pressure-sensitive adhesive according to any of the preceding claims, **characterized in that** the side chain functionalization moiety comprises at least two joint ring structures.

8. Pressure-sensitive adhesive according to any of the preceding claims, **characterized in that** the polyhydroxyalkanoate backbone has been prepared by fermentation.

## Patentansprüche

1. Druckempfindliches Haftmittel umfassend

   ein Polyhydroxyalkanoat, das **dadurch gekennzeichnet ist, dass** das Polyhydroxyalkanoat 1 bis 20 Gew.-% strukturelle Einheiten aufweist, die einen Seitenkettenfunktionalisierungsanteil aufweisen, der mindestens 10 C-Atome umfasst, wobei ein Seitenkettenfunktionalisierungsanteil eine chemische Gruppe ist, die durch weitere Funktionalisierung der ursprünglichen Seitenkette bereitgestellt wird, die von den Hydroxyalkansäuren stammt, auf denen das PHA formell basiert;
   wobei der Seitenkettenfunktionalisierungsanteil über ein Heteroatom an die Seitenkette gebunden ist.

2. Druckempfindliches Haftmittel nach Anspruch 1, das **dadurch gekennzeichnet ist, dass** das Heteroatom ein O-Atom ist.

3. Druckempfindliches Haftmittel nach einem der vorhergehenden Ansprüche, das **dadurch gekennzeichnet ist, dass** der Seitenkettenfunktionalisierungsanteil verzweigt ist.

4. Druckempfindliches Haftmittel nach einem der vorhergehenden Ansprüche, das **dadurch gekennzeichnet ist, dass** der Seitenkettenfunktionalisierungsanteil von einer natürlich vorkommenden Substanz abgeleitet ist.

5. Druckempfindliches Haftmittel nach einem der vorhergehenden Ansprüche , das **dadurch gekennzeichnet ist, dass** der Seitenkettenfunktionalisierungsanteil mindestens eine Ringstruktur umfasst.

6. Druckempfindliches Haftmittel nach einem der vorhergehenden Ansprüche, das **dadurch gekennzeichnet ist, dass** der Seitenkettenfunktionalisierungsanteil mindestens zwei C-Atome umfasst, die Chiralitätszentren sind.

7. Druckempfindliches Haftmittel nach einem der vorhergehenden Ansprüche, das **dadurch gekennzeichnet ist, dass** der Seitenkettenfunktionalisierungsanteil mindestens zwei gemeinsame Ringstrukturen umfasst.

8. Druckempfindliches Haftmittel nach einem der vorhergehenden Ansprüche, das **dadurch gekennzeichnet ist, dass** die Polyhydroxyalkanoat-Rückgratkette durch Fermentieren hergestellt worden ist.

**Revendications**

1. Adhésif sensible à la pression comprenant
un polyhydroxyalcanoate, **caractérisé en ce que** le polyhydroxyalcanoate présente 1 à 20 % en poids d'unités structurales ayant une fraction de fonctionnalisation de chaîne latérale comprenant au moins 10 atomes C, une fraction de fonctionnalisation de chaîne latérale étant un groupe chimique fourni par une autre fonctionnalisation de la chaîne latérale originale provenant des acides hydroxy alcane sur lesquels le PHA est formellement basé :
la fraction de fonctionnalisation de chaîne latérale étant liée à la chaîne latérale via un hétéroatome.

2. Adhésif sensible à la pression selon la revendication 1, **caractérisé en ce que** l'hétéroatome est un atome O.

3. Adhésif sensible à la pression selon l'une quelconque des revendications précédentes, **caractérisé en ce que** la fraction de fonctionnalisation de chaîne latérale est ramifiée.

4. Adhésif sensible à la pression selon l'une quelconque des revendications précédentes, **caractérisé en ce que** la fraction de fonctionnalisation de chaîne latérale est dérivée d'une substance naturelle.

5. Adhésif sensible à la pression selon l'une quelconque des revendications précédentes, **caractérisé en ce que** la fraction de fonctionnalisation de chaîne latérale comprend au moins une structure cyclique.

6. Adhésif sensible à la pression selon l'une quelconque des revendications précédentes, **caractérisé en ce que** la fraction de fonctionnalisation de chaîne latérale comprend au moins deux atomes C qui sont des centres de chiralité.

7. Adhésif sensible à la pression selon l'une quelconque des revendications précédentes, **caractérisé en ce que** la fraction de fonctionnalisation de chaîne latérale comprend au moins deux structures cycliques jointes.

8. Adhésif sensible à la pression selon l'une quelconque des revendications précédentes, **caractérisé en ce que** le squelette de polyhydroxyalcanoate a été préparé par fermentation.

FIG.1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019092118 A2

- US 20180186928 A1

**Non-patent literature cited in the description**

- **SATAS**. Handbook of Pressure Sensitive Adhesive Technology. 1999, 153-203
- *Appl. Microbiol. Biotechnol.*, January 2014, vol. 98 (2), 611-620

- Modification of polyhydroxyalkanoates. *RSG Green Chemistry*, January 2015, vol. 2015 (30), 141-182